# EUROPEAN PATENT SPECIFICATION

(11) **EP 4 536 130 B1**
(45) Date of publication and mention of the grant of the patent: **25.11.2026**
(21) Application number: 23736547.3
(22) Date of filing: 08.06.2023
(51) Int. Cl.: A61B 46/20, A61B 46/10, A61B 34/20, A61B 90/00, A61B 34/30

(54) **MEDICAL DRAPE WITH CONNECTOR SYSTEM FOR SURGICAL ROBOTIC TRACKING MARKER**
MEDIZINISCHES ABDECKTUCH MIT VERBINDERSYSTEM FÜR CHIRURGISCHEN ROBOTISCHEN VERFOLGUNGSMARKER
CHAMP OPÉRATOIRE MÉDICAL AVEC SYSTÈME DE CONNECTEUR POUR MARQUEUR DE SUIVI ROBOTISÉ CHIRURGICAL

(30) Priority: 11.06.2022 US 202263351341 P
(43) Date of publication of application: 16.04.2025
(73) Proprietor: Medline Industries, LP, Northfield, IL 60093 (US)
(72) Inventor: KLANN, Kenneth, James, Wilernie, Minnesota 55090 (US); WALD, Daniel, Robert, Burnsville, Minnesota 55337 (US)
(74) Representative: Lewis Silkin LLP
(86) International application number: PCT/US2023/024802
(87) International publication number: WO 2023/239843

(56) References cited:
- WO-A1-2011/038759
- WO-A1-2018/161057
- WO-A1-2019/010203

## Description

### TECHNICAL FIELD

This disclosure relates to sterile drape systems used to provide a sterile barrier during a medical procedure and, more particularly, to sterile drape systems for use with position recognition devices during robot-assisted surgery.

### BACKGROUND

Medical drapes, also referred to as surgical drapes, can be used during surgical procedures to help protect patients and equipment from contamination. For example, a patient drape may be used to cover portions of a patient's body not involved in a surgical procedure, providing a window or segregated space for accessing a region of the patient's body on which the surgical procedure is being performed. Such patient drapes may be used during a traditional surgical procedure performed under the direction of one or more clinicians or during a robot-assisted surgery. In the case of a robot-assisted surgical procedure, the surgeon may control a surgical robot that include one or more robotic arms manipulable by the surgeon during the surgical procedure. The surgical robot can include various medical instruments insertable into the patient for performing the surgical procedure and may also include visualization aids, such as endoscopes, cameras, etc. to observe what is happening in and/or around the patient at the site of the surgical procedure.

In robot-assisted surgeries, the location of the patient and objects, such as surgical instruments, typically need to be tracked with a high degree of precision as an instrument is being positioned and moved by a robot or by a physician. To do so, a position recognition systems can be used to determine the position of and track a particular object in 3-dimensions (3D). The position recognition system can use passive and/or active sensors or markers that are attached to an object, such as a patient, to be tracked. Objects to be tracked may include passive sensors, such as reflective spherical balls, which are positioned at strategic locations on the object to be tracked. When using active sensors or markers, the objects to be tracked include active infrared transmitters, such as light emitting diodes (LEDs), that generate their own signals for 3D detection. In either case, the system can geometrically resolve the 3- dimensional position of the active and/or passive sensors based on information from or with respect to one or more of the infrared cameras, digital signals, known locations of the active or passive sensors, distance, the time it took to receive the responsive signals, other known variables, or a combination thereof.

In practice, one or more tracking devices can be attached to a patient to track one or more anatomical locations of the patient during the surgical procedure. A tracking device may be desirably attached to the patient at a location covered by a surgical drape. If the tracking device is positioned under the surgical drape, the drape material can hinder accurate detection and tracking of the tracking device through the drape. However, if the tracking device is positioned outside of the surgical drape, the clinician may need to puncture the drape to provide an access hole for attaching the tracking device to the patient through the drape. Puncturing the drape can breach the sterility of the drape and undermine the effectiveness of the sterile draping.

WO 2019/010203 discloses a system and method of analysing and providing a patient's spinal alignment information and therapeutic device data. The system and/or method can obtain initial patient data and acquire spinal alignment contour information, and can assess localized anatomical features of the patient and obtain anatomical region data.

WO 2011/038759 discloses a medical tracking marker for localizing or tracking an object by means of a medical tracking system. This document also relates to a method of referencing an object using a medical navigation system.

WO 2018/161057 discloses a method and system for integrated diagnostic testing and real-time treatment that includes a medical data gathering device to capture multiple of source images, where at least one of the source images contains a fiducial marker. The method and system incorporate a low latency encoder to encode the captured source images into a data stream and further includes an environmental sensor device for the capturing of sensor data.

### SUMMARY

The invention provides a drape system according to claim 1. Further embodiments of the invention are provided in the dependent claims. In general, this disclosure is directed to connectors for connecting a patient tracking device to a patient through a drape in a medical procedure, including associated methods and systems involve such connectors and/or drapes. In some examples, a medical drape (also referred to as a surgical drape) defines a sheet of drape material configured (e.g., sized and/or shaped) to be positioned over at least a portion of a patient undergoing a medical procedure. The drape can include a region that defines a continuous sheet of material devoid of openings, which can define the entire extent of the drape or may be a part of the drape that also includes one or more openings (e.g., cutouts, windows) through which a surgeon can access the patient through the drape. In either case, a clinician may attach one or more patient tracking devices to the patient at a location on the patient to be covered by the drape. To connect the patient tracking device to the patient through the drape while helping to maintain the sterile barrier provided by the drape, a connector may be provided.

In some examples, a connector according to disclosure is configured to connect a patient tracking device across the thickness of the drape without creating a hole or otherwise breaching the drape. To do so in some implementations, the patient tracking device may be configured as a multipiece device that includes one component positionable under the drape and another component positionable over the drape. For example, the patient tracking device may include a base configured to be attached to the patient under the drape and a head configured to be positioned on an opposite side of the drape. The head of the patient tracking device can carry one or more tracking markers that can be tracked by a surgical position recognition system in the surgical environment.

The connector can also include multiple pieces positionable on opposite sides of the drape. For example, the connector can include a first connector body coupled to the base of the patient tracking device and a second connector body coupled to the head of the patient tracking device. The first connector body can connect to the second connector body with the drape material positioned between the two connector bodies. For instance, in some implementations, the first connector body and the second connector body may each magnets of opposite polarity from each other that are configured to magnetically attached to each other through the thickness of the drape material.

During use, the clinician can position the base of the patient tracking device at a target location on the patient. With the first connector body attached to the base of the patient tracking device, the clinician can position the drape over the patient, including over the first connector body. The clinician can then attach the second connector body to the first connector body with the drape sandwiched between the two connector bodies. The head of the patient tracking device can be coupled to the second connector body, e.g., before or after attaching the second connector body to the first connector body. The resulting set up can provide a patient that has a drape covering at least a portion of their body with a patient tracking device positioned external to the drape and connected to the patient at a location under the drape.

While a connector according to the disclosure can have a variety of different configurations as described herein, in some examples, the connector includes two connector bodies that attached to each other in a rotationally restricted position. For example, the two connector bodies may have corresponding geometric features that restrict rotational movement of the two connector bodies relative to each other when coupled together and/or sets of corresponding magnetic features that restrict rotational movement of the two connector bodies relative to each other when coupled together. In some implementations, the two connector bodies are configured to connect with each other in only a single rotational position. In other implementations, the two connector bodies are configured to connect with each other in multiple different rotational positions, although further rotational movement is inhibited once the two connector bodies are coupled together. Inhibiting rotational movement of the connector bodies relative to each other once coupled together may be useful to prevent inadvertent movement of one tracking body relative to the other tracking body during a surgical procedure, which may cause misalignment of one or more tracking markers relative to the intended location to be tracked.

In one example, a drape system is described that includes a drape and a connector. The drape is configured to be placed over at least a portion of a patient undergoing a medical procedure. The drape has a first side configured to face the patient and a second side opposite the first surface. The connector is configured to connect to a patient tracking device across the drape. The connector includes a first connector body configured to couple to a base of the patient tracking device and a second connector body configured to couple to a head of the patient tracking device. The base of the patient tracking device is configured to be attached to a patient and the head of the patient tracking device carries at least one tracking marker configured to be tracked by a surgical position recognition system. The example specifies that the first connector body is configured to connect to the second connector body with the drape positioned between the first connector body and the second connector body.

In another example, a drape system is described that includes a drape, a patient tracking device, and a connector. The drape is configured to be placed over at least a portion of a patient undergoing a medical procedure. The drape has a first side configured to face the patient and a second side opposite the first surface. The patient tracking device includes a base configured to be attached to a patient and a head carrying at least one tracking marker configured to be tracked by a surgical position recognition system. The base of the patient tracking device has a proximal end and the head of the patient tracking device has a distal end. The connector is configured to connect the patient tracking device across the drape. The connector includes a first connector body configured to couple to the proximal end of the base of the patient tracking device and a second connector body configured to couple to the distal end of the head of the patient tracking device. The example specifies that the first connector body is configured to connect to the second connector body with the drape positioned between the first connector body and the second connector body.

In another example, a connector for connecting a tracking device across a drape is described. The connector includes a first connector body and a second connector body. The first connector body is configured to couple to a base of a patient tracking device, the base of the patient tracking device being configured to be attached to a patient, and the first connector body defining a face configured to contact a first side of a drape for covering the patient. The second connector body is configured to couple to a head of the patient tracking device, the head of the patient tracking device carrying at least one tracking marker, the second connector body defining a face configured to contact a second side of a drape. The example specifies that the face of the first connector body is configured to connect to the face of the second connector body with the drape positioned between the first connector body and the second connector body.

In another example, a method of attaching a tracking device across a drape is described. The method includes positioning a first connector body attached to and/or attachable to a base of a tracking device on a first side of a drape. The method includes positioning a second connector body attached to and/or attachable to a head of the tracking device on a second side of the drape, the head carrying at least one tracking marker configured to be tracked by a surgical position recognition system. The method further includes connecting the first connector body to the second connector body with the drape positioned between the first connector body to the second connector body.

The details of one or more examples are set forth in the accompanying drawings and the description below. Other features, objects, and advantages will be apparent from the description and drawings, and from the claims.

### BRIEF DESCRIPTION OF DRAWINGS

FIG. 1 is an illustration of an example surgical environment that may utilize a connector and/or drape system according to the disclosure.
FIG. 2 is a perspective view of an example drape system that can be used in the example surgical environment of FIG. 1.
FIG. 3 is a perspective view of an example configuration of a connector that can be used in the drape system of FIG. 2.
FIG. 4 is a perspective view of an example connector body that can be used on the example connector of FIG. 2.
FIG. 5 is a diagram illustrating example magnetic arrays that may be used on the connector bodies of FIG. 2.

### DETAILED DESCRIPTION

This disclosure is generally directed to surgical drapes utilizing a connector system to attach a patient tracking device to a patient through the thickness of the drape while helping to maintain the structural integrity and sterile barrier provided by the drape. The connector system can include two bodies positionable on opposite sides of the drape with the thickness of the material forming the drape positioned between the two bodies. For example, the connector system can include a first body connected to and/or connectable to a first portion of the patient tracking device attachable to the patient. The connector system can also include a second body connected to and/or connectable to a second portion of the patient tracking device intended to be positioned external to the drape. The second portion of the patient tracking device can be or include one or more features detectable by a surgical position recognition system implemented in the operating environment where the patient is being draped. The two connector bodies can connect to each other physically and/or magnetically, thereby forming a joined connector with the drape extending through the cross-section of the connector.

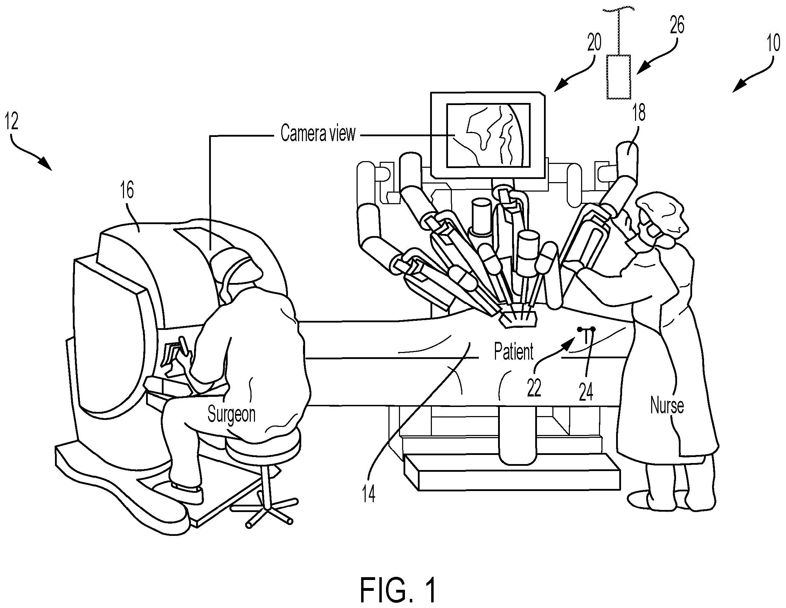

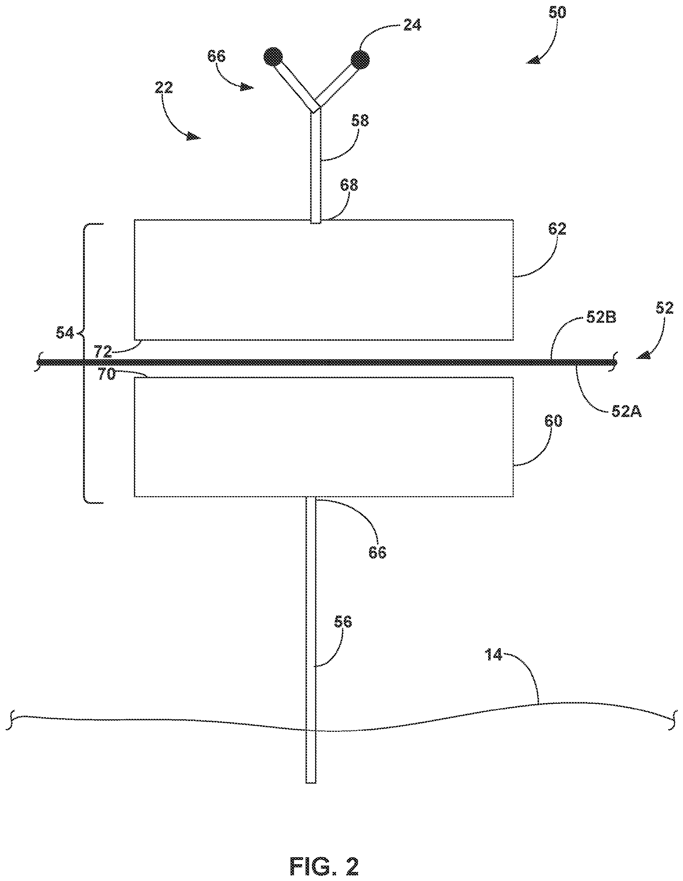

Example connector configurations will be described in greater detail with respect to FIGS. 2-5. However, an example surgical environment that may utilize a connector and/or drape system according to the disclosure will first be described with respect to FIG. 1.

FIG. 1 is an illustration of an example surgical environment that may utilize a connector and/or drape system according to the disclosure. In particular, FIG. 1 illustrates an example surgical environment 10 in which a robotic surgical system 12 is used to perform a surgical procedure on a patient 14. The example robotic surgical system 12 includes a surgeon control station 16, a patient-side robotic cart 18, and one or more displays 20. In use, a clinician can manipulate robotic surgical instruments carried by patient-side robotic cart 18 via surgeon control station 16 to perform a surgical procedure on patient 14. The one or more displays 20 can display electronic information concerning the surgical procedure being performed, such as images from inside body of the patient.

One or more patient tracking devices 22 may be attached to patient 14 to monitor the position / orientation of one or more locations of the patient of during the surgical procedure utilizing robotic surgical system 12. Each patient tracking device 22 may carry one or more tracking markers 24. A surgical position recognition system 26 can track the one or more tracking markers 24 carried by each tracking device 22. Addition tracking markers 24 may be attached to track the movement of a robot arm, an end-effector, and/or a surgical instrument in three dimensions in the surgical environment.

In some examples, surgical position recognition system 26 may be implemented using one or more cameras (e.g., mounted on a wall or ceiling surface of the surgical room and/or a movable camera stand). When so implemented, the camera may include any suitable camera or cameras, such as one or more infrared cameras (e.g., bifocal or stereophotogrammetric cameras), able to identify, for example, active and/or passive tracking markers 24 in a given measurement volume viewable from the perspective of the camera. The camera may scan the given measurement volume and detect the light that comes from the markers 24 in order to identify and determine the position of the markers 24 in three-dimensions. For example, active markers 24 may include infrared-emitting markers that are activated by an electrical signal (e.g., infrared light emitting diodes (LEDs)), and passive markers 24 may include retro-reflective markers that reflect infrared light (e.g., reflect incoming IR radiation into the direction of the incoming light), for example, emitted by illuminators on the camera or other suitable device.

In some examples, a plurality of tracking markers 24 can be mounted (or otherwise secured) thereon to an outer surface of the robot, such as, for example, on the base of robot, on the robot arm 104, or on the end-effector of the robot. One or more tracking markers 24 can further be mounted (or otherwise secured) to patient 14 as described herein. In some examples, the plurality of tracking markers 24 can be positioned on the patient 14 spaced apart from the surgical field to reduce the likelihood of being obscured by the surgeon, surgical tools, or other parts of the robot. Further, one or more tracking markers 24 can be further mounted (or otherwise secured) to the surgical tools (e.g., a screw driver, dilator, implant inserter, or the like). Thus, the tracking markers can enable each of the marked objects to be tracked by the robot. In examples, the system can use tracking information collected from each of the marked objects to calculate the orientation and location of the tracked object relative to the relative position of patient 14.

Tracking markers 24 may include radiopaque or optical markers. Tracking markers 24 may be suitably shaped include spherical, spheroid, cylindrical, cube, cuboid, or the like. In some examples, one or more of tracking markers 24 may be optical markers. For example, tracking markers 24 may be infrared light-emitting diodes (LEDs) capable of being tracked using an infrared optical tracking system. Additionally or alternatively, tracking markers 24 can comprise conventional reflective spheres capable of being tracked using an optical tracking system. In some implementations, tracking markers 24 attached to patient 16 are passive reflective markers, such as passive reflective spheres. In either case, light emitted from and/or reflected by tracking markers 24 can be detected by surgical position recognition system 26 (which may be implemented using one or more cameras), and can be used to monitor the location and movement of the marked objects. Tracking markers 24 may be sight devices. Additionally or alternatively, tracking markers 24 can comprise a radio-frequency and/or electromagnetic reflector or transceiver and surgical position recognition system 26 can include or be replaced by a radio-frequency and/or electromagnetic transceiver.

One or more computing devices associated with surgical position recognition system 26 may receive and process information from other components in order to display information to the user. For example, one or more computing devices associated with surgical position recognition system 26 may track the location of certain markers that are located on the different components of system and/or instruments used by a clinician during a surgical procedure relative to the location of the patient also tracked by the system. The location, orientation, and/or position of structures having tracking markers may be provided to the one or more computing devices, which may be displayed to a clinician on display 20 or another display device. For example, a surgical instrument may be shown to a user in relation to a three dimensional image of a patient's anatomical structure.

FIG. 2 is a perspective view of an example drape system 50 that can be used in the example surgical environment of FIG. 1. Drape system 50 includes a drape 52 configured to be placed over at least a portion of patient 14 undergoing a medical procedure. Drape system 50 can also include one or more patient tracking devices 22 configured to be attached to patient 14. The one or more patient tracking devices 22 can each carry one or more tracking markers 24 configured to be tracked by surgical position recognition system 26 (FIG. 1). A connector 54 can connect patient tracking device 22 across drape 52 while maintaining the sterile barrier provided by the drape. For example, connector 54 may connect patient tracking device 22 across the thickness of drape 52 without puncturing the drape material or requiring a preformed cut out in the drape material through which patient tracking device 22 passes.

In the illustrated arrangement of FIG. 2, patient tracking device 22 is illustrated as being formed of multiple separate pieces configured to be positioned on opposite sides of drape 52. In particular, patient tracking device 22 is illustrated as including a base 56 and a head 58. Base 56 is configured to be attached to patient 14. Head 58 can carry one or more tracking markers 24. Base 56 of patient tracking device 22 can be positioned on a first side 52A of drape 52 facing patient 14 (with the base extending between the patient and the drape). Head 58 of patient tracking device 22 can be positioned on a second side 52B of drape 52 opposite the first side 52A of the drape. Accordingly, head 58 of patient tracking device 22 and the one or more tracking markers 24 carried by the head can be outside of the space covered by drape 52 for detection by surgical position recognition system 26.

Connector 54 can connect patient tracking device 22 across drape 52. Connector 54 may include at least two separable pieces that her joinable together to define the connector: one piece positionable on the first side 52A of drape 52 and a second piece positionable on the second side 52B of the drape. In the illustrated arrangement, connector 54 is shown implemented using a first connector body 60 and a second connector body 62First connector body 60 can be coupled to base 56 of patient tracking device 22. Second connector body 62 can be coupled to head 58 of patient tracking device 22. First connector body 60 is configured to mate with and/or connect to second connector body 62 with drape 52 position between the two connector bodies. When connected together, first connector body 60 and second connector body 62 may be temporarily interlocked such that the two connector bodies are configured to not detach in the absence of a deliberate detaching action by the clinician. FIG. 2 illustrates first connector body 60 separated from second connector body 62 with the drape 52 interposed between the two connector bodies, with the two connector bodies being engageable together to trap the drape between the two connector bodies.

First connector body 60 and second connector body 62 can be configured a variety of different ways to allow the two components to form a connection across the thickness of drape 52. In some implementations, each connector body includes one or more magnets allowing the two connector bodies to magnetically connect to each other across the thickness of drape 52. Additionally or alternatively, each connector body may be configured to physically engage with and/or mechanically interlock with the other connector body to form a connection between the two connector bodies. For example, one connector body may include one or lips, recesses, barbs, tangs, projections, cavities that are configured to engage with (e.g., interlock with) one or complementary and/or corresponding structures on the other connector body with drape 52 interposed between the two connector bodies. Drape 52 may bend, fold, and/or otherwise deform around and/or conform to the size and shape of the interconnection features between the two connector bodies.

Independent of the specific means by which first connector body 60 and second connector body 62 are configured to connect to each other, the two connector bodies may connect to each other with drape 52 positioned between the two connector bodies without piercing the drape. For example, the section of drape 52 positioned between first connector body 60 and second connector body 62 may be a region of continuous drape material devoid of openings extending through the thickness of the drape. First connector body 60 and second connector body 62 can be connected together with drape 52 position between the two connector bodies while maintaining the structural integrity of the drape and the barrier properties provided by the drape. After detaching the two connector bodies from each other after use, the section of drape material previously positioned between the two connector bodies may remain devoid of openings (e.g., punctures, piercings). This can help maintain the sterile barrier provided by the drape when using connector 54.

That being said, in other examples, one connector body may include one or more features configured to puncture through the thickness of drape 52, e.g., and interconnect with one or more complementary features on the other connector body. The connection between the two connector bodies may be sufficiently tight after puncturing drape 52 during interconnection of the two connector bodies that connector 54 occludes the puncture(s) in the resulting assembly provides a sterile barrier for the remainder of the surgical procedure.

Base 56 of patient tracking device 22 may be implemented using any structure configured to directly or indirectly attached to patient 14. Base 56 may be attached to a rigid anatomy of patient 14 and may remain attached throughout the surgical procedure. For example, base 56 may attach to a bone of the patient, such as a bone that is located away from the targeted anatomical structure subject to the surgical procedure. Base 56 may define a shaft configured to be inserted into patient 14. For example, base 56 may define a shaft configured to be inserted percutaneously through the skin of patient 14 into an underlying bone. In other examples, base 56 may attach external to the skin of patient 14.

Head 58 of patient tracking device 22 may be implemented using any structure configured to directly or indirectly attached tracking markers 24. In some examples, head 58 defines a shaft extending outwardly (e.g., orthogonally) from the underlying body of patient 14. The one or more tracking markers 24 can be positioned at any desired location along the length of head 58, including a top surface and/or a side surface of the head.

In some implementations, head 58 defines a tracking array 64 carrying a plurality of tracking markers 24. For example, an array of two, three, four or more tracking markers 24 may be provided on head 58 of patient tracking device 22. Tracking array 64 may include a linear section, a cross piece, and may be asymmetric such that the different tracking markers are at different relative positions and locations with respect to one another on the tracking array.

Base 56 may define a fixed orientation relative to first connector body 60 or may define an adjustable orientation relative to the connector body. Similarly, head 58 may define a fixed orientation relative to second connector body 62 or may define an adjustable orientation relative the connector body. When base 56 and/or head 58 are configured with an adjustable orientation, the clinician may adjust the orientation of the feature relative to the corresponding connector body and then lock the adjusted orientation to hold the position. This can provide flexibility for the clinician to adjust the location of one or more tracking markers 24 after connecting first connector body 60 to second connector body 62.

Patient tracking device 22, including base 56 and head 58, may be sterilizable and reusable multiple times across different patients. When so configured, base 56 may or may not be detachably couplable to first connector body 60 and/or head 58 may or may not be detachably couplable two second connector body 62. Connector 54 may be a single use component intended to be disposed after a single use or, instead, may be a sterilizable connector intended to be reused multiple times with a patient tracking device.

In one implementation, connector 54 is a single use component included with each new drape 52. The clinician can obtain a reusable patient tracking device 22, connect the constituent components of the patient tracking device to connector 54, and use the resulting assembly during a surgical procedure with drape 52. After the surgical procedure, the clinician can detach the constituent components of patient tracking device 22 from connector 54, dispose drape 52 and connector 54, and patient tracking device 22 can be sterilized.

In another implementation, connector 54 may be reusable with patient tracking device 22. When so configured, the constituent components of patient tracking device 22 may or may not be detachable from the constituent components of connector 54. For example, base 56 may be permanently attached to first connector body 60 (e.g., to form a unitary structure not intended to be detached from each other) or the base may be detachably attached to the first connector body (e.g., to form a unitary structure that is intended to be separated into two or more individual components from each other). Likewise, head 58 may be permanently attached to second connector body 62 (e.g., to form a unitary structure not intended to be detached from each other) or the base may be detachably attached to the second connector body (e.g., to form a unitary structure that is intended to be separated into two or more individual components from each other).

Independent of whether base 56 and head 58 are permanently or detachably coupled to first connector body 60 and second connector body 62, respectively, the base and head may be joined to the connector bodies in such a way as to configure the connector bodies to engage with each other. For example, base 56 may define a proximal end 66 to which first connector body 60 is coupled. Head 58 may define a distal end 68 two which second connector body 62 is coupled. A variety of mechanical connection features can be utilized to couple base 56 and head 58 to first connector body 60 and second connector body 62, respectively. For example, screws, pins, adhesive, friction fit, and/or another feature may be used to connect one component of patient tracking device 22 to a corresponding component of connector 54. Alternatively, the component of patient tracking device 22 may be formed with the corresponding component of connector 54 (e.g., by casting, molding, milling, or the like).

In use, first connector body 60 and second connector body 62 can be engaged with each other with drape 52 sandwich between the two connector bodies. For example, first connector body 60 can define a face 70 configured to contact the first side 52A of drape 52. Second connector body 62 can define a face 72 configured to contact the second side 52B of drape 52. Face 70 of first connector body 60 may have a complementary shape to face 72 of second connector body 62. For example, the profile of face 70 of first connector body 60 (e.g., surface features, depth change across the face) may conform to the profile of face 72 of second connector body 62. In one example, face 70 of first connector body 60 has a profile that is a mirror image (e.g., substantially equal and opposite of) the profile of face 72 of second connector body 62. In other examples, face 70 of first connector body 60 has a different profile than the profile of face 72 of second connector body 62, but the two profiles are sufficiently similar and/or indexed relative to each other to allow the two connector bodies to interconnect with each other.

In the illustrated arrangement of FIG. 2, face 70 of first connector body 60 and face 72 of second connector body 62 are each illustrated as defining planar faces. When so configured, drape 52 can be sandwich between the flat faces of first connector body 60 and second connector body 62. Face 70 of first connector body 60 and/or face 72 of second connector body 62 may or may not include adhesive (e.g., pressure sensitive adhesive) to help fix the connector body to drape 52. In some configurations, first connector body 60 and/or second connector body 62 may define a nonplanar face. The nonplanar face may be characterized by having one or more recesses and/or projections extending in a dimension parallel to an axis defined by the thickness of drape 52.

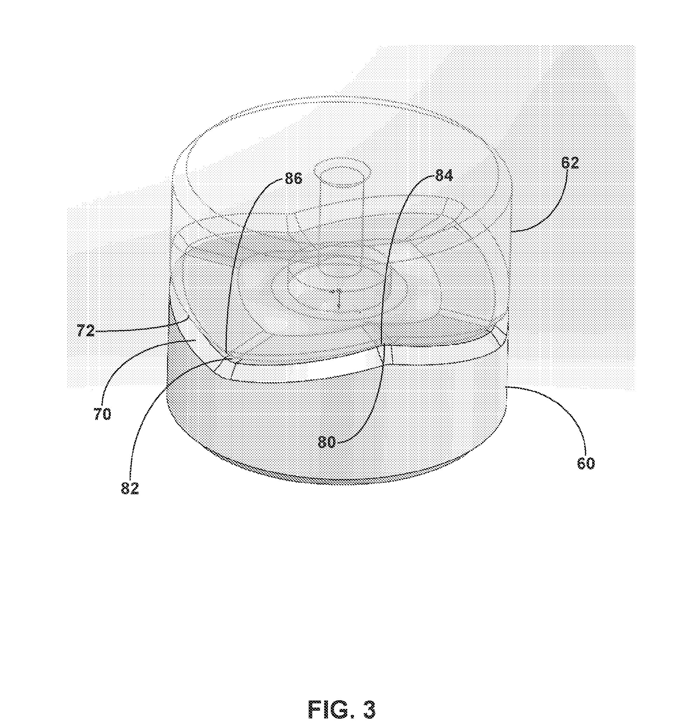

FIG. 3 is a perspective view of an example configuration of connector 54 with first connector body 60 and second connector body 62 having nonplanar faces 70, 72. When configured with nonplanar faces, drape 52 positioned between the two connector bodies after assembly may be bent or deformed to be positioned in different planes extending across the connector. This may help prevent relative movement between connector 54 and drape 52 after assembly.

As shown in the example of FIG. 3, face 70 of first connector body 60 may include one or more projections 80 and/or recesses 82. Face 72 of second connector body 62 may include one or more complementary recesses 84 and/or projections 86 configured to fit into the one or more projections 80 and/or recesses 82 defined on the face of the first connector body. In some examples, face 70 of first connector body 60 includes a plurality of alternating projections 80 and recesses 82 arrayed around the face of the connector body (e.g., around the perimeter or circumference of the connector body). Face 72 of second connector body 62 may include a plurality of complementary recesses 84 and projections 86 arrayed around the face of the connector body (e.g., around the perimeter or circumference of the connector body) and configured to fit into the alternating projections and recesses of the first connector body. The projection and/or recess on each of first connector body 60 and second connector body 62 can have any desired shape (e.g., defined by a sharp angle, curved radius) in any desired height or depth.

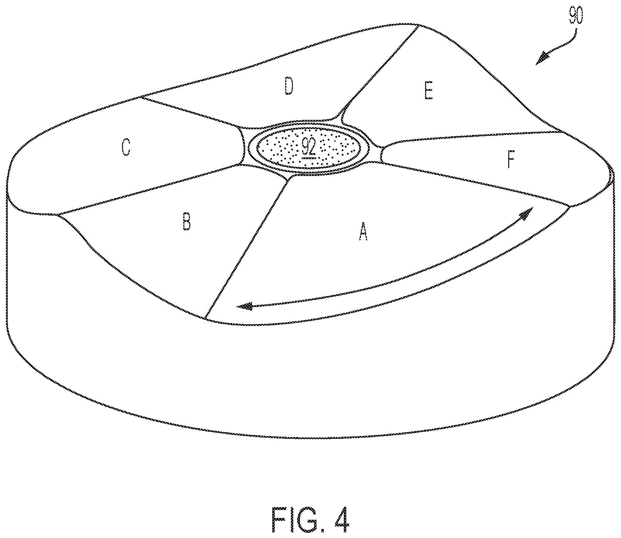

FIG. 4 is a perspective view of an example connector body 90 that can be used on connector 54 of FIG. 2. Connector body 90 can be representative of first connector body 60 and/or second connector body 62, with the corresponding connector body having a complementary profile. As shown in this example, connector body 90 includes a magnet 92 for magnetically coupling the connector body to the corresponding connector body with drape 52 interposed between the two connector bodies. For example, first connector body 60 may carry a first magnet, second connector body 62 may carry a second magnet, and the two connector bodies may magnetically attach to each other via magnetic attraction of the two magnets. In the illustrated arrangement, magnet 92 is illustrated as being positioned at approximately a geometric center of connector body 90 (e.g., ± 10% from the geometric center). In other configurations, magnet 92 may be offset from the geometric center of the connector body.

With further reference to FIG. 3, first connector body 60 may be configured to connect to second connector body 62 with the first connector body being rotationally fixed relative to the second connector body. For example, configuring the two connector bodies with corresponding projections and/or recesses may cause the two connector bodies to move into a particular rotational alignment when fitting corresponding projections and recesses of the two connector bodies into each other during assembly. In some configurations, the two connector bodies can be rotationally fixed to each other in multiple different rotational positions. For example, as illustrated in FIG. 3, the different projections and recesses defined by first connector body 60 and second connector body 62 have a same size and/or shape. This allows one connector body to be rotated until any projection is aligned with any recess of the corresponding connector body.

In other configurations, first connector body 60 and second connector body 62 may be rotationally fixed relative to each other in only a single rotational position. When so configured, face 70 of first connector body 60 and face 72 of second connector body 62 may need to be rotated relative to each other until the two faces are in one particular relative orientation with respect to each other, with the two faces not indexing with each other in other relative orientations. Such an arrangement can be useful to provide a fixed orientation of head 58, including the one or more tracking markers 24 carried by the head, relative to base 56 attach the patient. For example, if the clinician wants or needs to remove head 58 by detaching second connector body 62 from first connector body 60, the clinician do so and can subsequently reattach the two connector bodies together and position head 58 in the same relative orientation as when the head was removed.

A variety of different features can be implemented to configure first connector body 60 to connect a second connector body 62 in a single rotational orientation position. With further reference to FIG. 4, for example, the face of each connector body may have an asymmetric profile. For example, the face of each connector body may have one or more projections and/or recesses that are configured differently (e.g., have a different size and/or shape) than each other projection and/or recess defined on the face of the connector body. In the illustrated example, for instance, the face of the connector body is shown as having multiple facets (e.g., six as illustrated) that are configured differently from each other. The other connector body can have a complementary profile. When configured with an asymmetric profile, first connector body 60 and second connector body 62 may connect with each other in a single rotational position. In practice, it should be appreciated that the two connector bodies may or may not couple together (e.g., magnetically) when misaligned with the single rotational position when the surface features on the two faces are aligned. However, the clinician may detect the misalignment by observing a gap between edges of the two components and/or tactile feel when rotationally allying the two components to determine if the two components are or are not in their single target rotational position.

In addition to or in lieu of configuring the face of first connector body 60 in the face of second connector body 62 to align in one or more specific rotational positions, the connector bodies are configured with one or more magnetics that are arranged to control the rotational positioning of the two components relative to each other. For example, when first connector body 60 and second connector body 62 each include a magnet radially offset from the geometric center of the connector bodies, the corresponding magnets may align with each other to form a magnetic connection only when the connector bodies are in a specific rotational position.

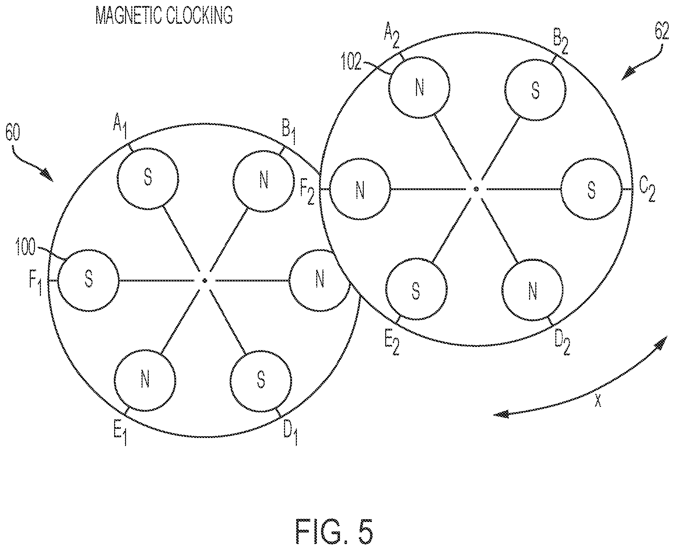

First connector body 60 and/or second connector body 62 include a set of magnets that are positioned to control the relative rotational alignment between the two connector bodies. FIG. 5 is a diagram illustrating example magnetic arrays that may be used on the connector bodies of connector 54 of FIG. 2. As shown in this example, first connector body 60 includes a first set of magnets 100 with at least one magnet in the first set of magnets having a different polarity than at least one other magnet in the first set of magnets. The polarity of the maintenance are represented as north "N" and south "S" poles on the illustrated diagram. Second connector body 62 includes a second set of magnets 102 with at least one magnet in the second set of magnets having a different polarity than at least one other magnet in the second set of magnets. First set of magnets 100 and set of magnets 102 may each be implemented utilizing any suitable number of magnets (e.g., two, three, four, five, six or more). In either case, the polarities of the different magnets in the first set of magnets 100 may be arranged relative to the polarities of the different magnets and the second set of magnets 102 such that, when the clinician places first connector body 60 and second connector body 62 together to connect the two bodies, the magnetic attraction/repulsion forces dictate the rotational alignment of the two bodies relative to each other.

First connector body 60 and/or second connector body 62 may have any arcuate (e.g., circular, oval, elliptical) or polygonal (e.g., triangle, square, pentagon, hexagon, octagon) shape, or combinations of arcuate and polygonal shapes. While first connector body 60 may typically be configured to have the same shape as second connector body 62, the two components may have different shapes provided an interlocking connection can be made between the two components. Each connector body may be formed of a polymeric material (e.g., plastic), metal, ceramic, and/or other suitable material.

In general, drape 52 can be made of a sterile (and/or sterilizable) material that can be disposed of after a single use. Drape 52 can be fabricated from any suitable material or combinations of materials. For example, drape 52 may be fabricated from a polymeric material, a fiber cloth (e.g., woven or nonwoven cloth, such as cotton cloth), and/or paper. Example types of polymeric materials that may be used to fabricate drape 52 include, but are not limited to, polyvinyl chloride, polyethylene, polypropylene, polyurethane, polystyrene, and/or polycarbonate. After manufacture but before use, drape 52 may or may not be sterilized to ensure that the drape provides a sterile barrier over patient 14. For example, drape 52 may be subject to a sterilization process, such as steam sterilization, dry heat sterilization, ethylene oxide gas sterilization, or radiation sterilization. Connector 54 may also be sterilized using any of the mentioned sterilization processes.

In some applications, drape 52 and connector 54 are provided as a kit. For example, drape 52 and connector 54 may be provided in a bag or other sterile container. In use, the clinician can open the sterile container, remove connector 54 for use as described herein, and also remove drape 52 to be placed over a patient undergoing a medical procedure. While the preceding discussion has focused on a single patient tracking device 22 and single connector 54, in practice, it should be appreciated that multiple patient tracking devices and multiple connectors 54 may be provided and/or used with drape 52 without departing from the scope of disclosure. Accordingly, any suitable number of connectors (e.g., one, two, three, four, or more) may optionally be provided as part of a kit including drape 52.

In addition, although tracking device 22 has generally been described as a patient tracking device and drape 52 has generally been described as a patient drape, it should be appreciated that the concepts of the disclosure may also be used for nonpatient applications. For example, tracking device 22 may be attached to medical equipment (e.g., a portion of robotic surgical system 12, such as an arm of the robotic surgical system) that is desirably covered by an equipment drape. In these configurations, base 56 of tracking device 22 can be configured to attach, directly or indirectly, to any desired piece of equipment, and drape 52 may be configured to drape any such desired piece of equipment. Accordingly, reference to a patient in the preceding description may be replaced with a corresponding reference to the one or more pieces of surgical equipment or other hardware to be draped and tracked utilizing the concepts described herein.

In use, a clinician can use connector 54 to attach a tracking device 22 across drape 52. The clinician can position first connector body 60 on a first side of drape 52. First connector body 60 can be attached to and/or attachable to base 56 of tracking device 22 (e.g., permanently attached to the base or attached to the base before or after assembly of the connector to the drape). Before or after, the clinician can position second connector body 62 on a second side of drape 52. Second connector body 62 can be attached to and/or attachable to head 58 (e.g., permanently attached to the head or attached to the head before or after assembly of the connector to the drape). The clinician can then interconnect first connector body 60 with second connector body 62.

In some examples, the clinician moves the two connector bodies toward each other (e.g., moves second connector body 62 axially toward first connector body). When configured with magnetic coupling, the two connector bodies may interconnect with each other when the corresponding magnets carried by the two bodies are sufficiently close to each other and/or in appropriate rotational alignment. The clinician may rotate one connector body relative to the other connector body to find an appropriate relative location of the two bodies where physical and/or magnetic alignment exists. In some examples, the clinician presses and/or squeezes the two bodies together. For example, when forming a mechanical interconnection between the two bodies without magnetic coupling, the clinician may press the two connector bodies together to form a mechanical coupling between the two bodies.

Independent of the specific configuration, a connector system according to the disclosure can provide an efficient and effective mechanism for connecting a tracking device across a drape while helping to maintain the sterility and integrity of the drape.

Various examples have been described. These and other examples are within the scope of the following claims.

## Claims

1. A drape system (50) comprising:
a drape (52) configured to be placed over at least a portion of a patient undergoing a medical procedure, the drape having a first side (52A) configured to face the patient and a second side (52B) opposite the first surface; and
a connector (54) configured to connect to a patient tracking device (22) across the drape, the connector comprising a first connector body (60) configured to couple to a base (56) of the patient tracking device and a second connector body (62) configured to couple to a head (58) of the patient tracking device, wherein the base of the patient tracking device is configured to be attached to a patient and the head of the patient tracking device carries at least one tracking marker configured to be tracked by a surgical position recognition system, **characterized in that**:
the first connector body is configured to connect to the second connector body with the drape positioned between the first connector body and the second connector body;
the first connector body is configured to connect to the second connector body with the first connector body being rotationally fixed relative to the second connector body;
the first connector body comprises a first set of magnets (100) with at least one magnet in the first set of magnets having a different polarity than at least one other magnet in the first set of magnets;
the second connector body comprises a second set of magnets (102) with at least one magnet in the second set of magnets having a different polarity than at least one other magnet in the second set of magnets; and
the first connector body is configured to connect to the second connector body with the first connector body rotationally aligned with the second connector body in a specific rotational position so the first set of magnets magnetically couples to the second set of magnets such that attraction/repulsion forces between the first set of magnets and the second set of magnets dictate the rotational alignment of the first connector body and the second connector body.

2. The drape system of claim 1, wherein:
the first connector body has a face (70) configured to contact the first side of the drape;
the second connector body has a face (72) configured to contact the second side of the drape; and
the face of the first connector body has a complementary shape to the face of the second connector body.

3. The drape system of claim 2, wherein the face of the first connector body and the face of the second connector body are each planar.

4. The drape system of claim 2, wherein the face of the first connector body and the face of the second connector body are each non-planar such that, when the drape is positioned between the first connector body and the second connector body, the drape material is bent in different planes extending across the connector.

5. The drape of claim 4, wherein:
the face of the first connector body includes one or more projections (80) and/or recesses; and
the face of the second connector body includes one or more complementary recesses (82) and/or projections configured to fit into the one or more projections and/or recesses defined on the face of the first connector body.

6. The drape system of claim 4 or 5, wherein:
the face of the first connector body includes a plurality of alternating projections and recesses; and
the face of the second connector body includes a plurality of complementary recesses and projections configured to fit into the alternating projections and recesses of the first connector body.

7. The drape system of any one of claims 1 to 6, wherein the head of the patient tracking device comprises a shaft, and the distal end of the head is defined by a distal end of the shaft.

8. The drape system of any one of claims 1 to 7, wherein the head of the patient tracking device comprises a tracking array (64) carrying a plurality of tracking markers (24).

9. The drape system of any one of claims 1 to 8, wherein the base of the patient tracking device comprises a shaft, and the proximal end of the base is defined by a proximal end of the shaft.

10. The drape system of claim 9, wherein the shaft is configured to be inserted into the patient.

11. The drape system of claim 10, wherein the shaft is configured to be inserted percutaneously into a bone of the patient.

12. The drape system of any one of claims 1 to 11, wherein the surgical position recognition system comprises an optical surgical position recognition system.

13. The drape system of any one of claims 1 to 12, wherein the drape material comprises a polymeric material.

14. The drape system of any one of claims 1 to 13, wherein the drape material defines a continuous sheet of material devoid of any apertures in the region between the first connector body and the second connector body.

## Patentansprüche

1. Drape-System (50), umfassend:
ein Drape (52), das so konfiguriert ist, dass es mindestens einen Teil eines Patienten abdeckt, der sich einem medizinischen Eingriff unterzieht, wobei das Drape eine erste Seite (52A), die so konfiguriert ist, dass sie dem Patienten zugewandt ist, und eine der ersten Seite gegenüberliegende zweite Seite (52B) aufweist; und
ein Verbindungselement (54), das so konfiguriert ist, dass es über das Drape hinweg mit einer Patienten-Tracking-Vorrichtung (22) verbunden wird, wobei das Verbindungselement einen ersten Verbindungskörper (60) umfasst, der so konfiguriert ist, dass er mit einer Basis (56) der Patienten-Tracking-Vorrichtung gekoppelt wird, und einen zweiten Verbindungskörper (62) umfasst, der so konfiguriert ist, dass er mit einem Kopf (58) der Patienten-Tracking-Vorrichtung gekoppelt wird, wobei die Basis der Patienten-Tracking-Vorrichtung so konfiguriert ist, dass sie an einem Patienten befestigt wird, und der Kopf der Patienten-Tracking-Vorrichtung mindestens einen Tracking-Marker trägt, der so konfiguriert ist, dass er von einem chirurgischen Positionserkennungssystem verfolgt wird, **dadurch gekennzeichnet, dass**:
der erste Verbindungskörper so konfiguriert ist, dass er mit dem zweiten Verbindungskörper verbunden wird, wobei das Drape zwischen dem ersten Verbindungskörper und dem zweiten Verbindungskörper angeordnet ist;
der erste Verbindungskörper so konfiguriert ist, dass er mit dem zweiten Verbindungskörper verbunden wird, wobei der erste Verbindungskörper drehbar im Verhältnis zu dem zweiten Verbindungskörper befestigt ist;
der erste Verbindungskörper einen ersten Satz von Magneten (100) umfasst, wobei mindestens ein Magnet des ersten Satzes von Magneten eine andere Polarität aufweist als mindestens ein anderer Magnet in dem ersten Satz von Magneten;
der zweite Verbindungskörper einen zweiten Satz von Magneten (102) umfasst, wobei mindestens ein Magnet des zweiten Satzes von Magneten eine andere Polarität aufweist als mindestens ein anderer Magnet in dem zweiten Satz von Magneten; und
der erste Verbindungskörper so konfiguriert ist, dass er mit dem zweiten Verbindungskörper verbunden werden kann, wobei der erste Verbindungskörper in einer bestimmten Drehstellung relativ zum zweiten Verbindungskörper drehbar ausgerichtet ist, sodass der erste Satz von Magneten magnetisch mit dem zweiten Satz von Magneten gekoppelt wird, wobei Anziehungs-/Abstoßungskräfte zwischen dem ersten Satz von Magneten und dem zweiten Satz von Magneten die Drehausrichtung des ersten Verbindungskörpers und des zweiten Verbindungskörpers zueinander bestimmen.

2. Drape-System nach Anspruch 1, wobei:
der erste Verbindungskörper eine Fläche (70) aufweist, die so konfiguriert ist, dass sie mit der ersten Seite des Drapes in Kontakt kommt;
der zweite Verbindungskörper eine Fläche (72) aufweist, die so konfiguriert ist, dass sie mit der zweiten Seite des Drapes in Kontakt kommt; und
die Fläche des ersten Verbindungskörpers eine zur Fläche des zweiten Verbindungskörpers komplementäre Form aufweist.

3. Drape-System nach Anspruch 2, wobei die Fläche des ersten Verbindungskörpers und die Fläche des zweiten Verbindungskörpers jeweils eben sind.

4. Drape-System nach Anspruch 2, wobei die Fläche des ersten Verbindungskörpers und die Fläche des zweiten Verbindungskörpers jeweils nicht eben sind, sodass das Drape-Material, wenn das Drape zwischen dem ersten Verbindungskörper und dem zweiten Verbindungskörper angeordnet ist, in unterschiedlichen Ebenen gebogen wird, die sich über den Verbindungskörper erstrecken.

5. Drape nach Anspruch 4, wobei:
die Fläche des ersten Verbindungskörpers einen oder mehrere Vorsprünge (80) und/oder Vertiefungen einschließt; und
die Fläche des zweiten Verbindungskörpers eine oder mehrere komplementäre Vertiefungen (82) und/oder Vorsprünge einschließt, die so konfiguriert sind, dass sie in den/die eine/n oder die mehreren Vorsprünge und/oder Vertiefungen eingreifen, die an der Fläche des ersten Verbindungskörpers ausgebildet sind.

6. Drape-System nach Anspruch 4 oder 5, wobei:
die Fläche des ersten Verbindungskörpers eine Vielzahl von abwechselnd angeordneten Vorsprüngen und Vertiefungen einschließt;
und die Fläche des zweiten Verbindungskörpers eine Vielzahl von komplementären Vertiefungen und Vorsprüngen einschließt, die so konfiguriert sind, dass sie in die abwechselnd angeordneten Vorsprünge und Vertiefungen des ersten Verbindungskörpers eingreifen.

7. Drape-System nach einem der Ansprüche 1 bis 6, wobei der Kopf der Patienten-Tracking-Vorrichtung einen Schaft umfasst und das distale Ende des Kopfes durch ein distales Ende des Schafts gebildet ist.

8. Drape-System nach einem der Ansprüche 1 bis 7, wobei der Kopf der Patienten-Tracking-Vorrichtung eine Tracking-Anordnung (64) umfasst, die eine Vielzahl von Tracking-Markern (24) trägt.

9. Drape-System nach einem der Ansprüche 1 bis 8, wobei die Basis der Patienten-Tracking-Vorrichtung einen Schaft umfasst und das proximale Ende der Basis durch ein proximales Ende des Schafts gebildet ist.

10. Drape-System nach Anspruch 9; wobei der Schaft so konfiguriert ist, dass er in den Patienten eingesetzt wird.

11. Drape-System nach Anspruch 10, wobei der Schaft so konfiguriert ist, dass er perkutan in einen Knochen des Patienten eingesetzt wird.

12. Drape-System nach einem der Ansprüche 1 bis 11, wobei das chirurgische Positionserkennungssystem ein optisches chirurgisches Positionserkennungssystem umfasst.

13. Drape-System nach einem der Ansprüche 1 bis 12, wobei das Drape-Material ein polymeres Material umfasst.

14. Drape-System nach einem der Ansprüche 1 bis 13, wobei das Drape-Material eine durchgehende Materialbahn definiert, die in dem Bereich zwischen dem ersten Verbindungskörper und dem zweiten Verbindungskörper frei von Öffnungen ist.

## Revendications

1. Système de champ opératoire (50) comprenant :
un champ opératoire (52) conçu pour être placé sur au moins une partie d'un patient subissant un acte médical, le champ opératoire présentant un premier côté (52A) conçu pour faire face au patient et un second côté (52B) opposé à la première surface ; et
un connecteur (54) conçu pour se connecter à un dispositif de suivi de patient (22) à travers le champ opératoire, le connecteur comprenant un premier corps de connecteur (60) conçu pour se coupler à une base (56) du dispositif de suivi de patient et un second corps de connecteur (62) conçu pour se coupler à une tête (58) du dispositif de suivi de patient, dans lequel la base du dispositif de suivi de patient est conçue pour être fixée à un patient et la tête du dispositif de suivi de patient porte au moins un marqueur de suivi conçu pour être suivi par un système de reconnaissance de position chirurgical, **caractérisé en ce que** :
le premier corps de connecteur est conçu pour se connecter au second corps de connecteur, le champ opératoire étant positionné entre le premier corps de connecteur et le second corps de connecteur.
le premier corps de connecteur est conçu pour se connecter au second corps de connecteur, le premier corps de connecteur étant fixe en rotation par rapport au second corps de connecteur.
le premier corps de connecteur comprend un premier ensemble d'aimants (100), au moins un aimant du premier ensemble d'aimants présentant une polarité différente de celle d'au moins un autre aimant du premier ensemble d'aimants.
le second corps de connecteur comprend un second ensemble d'aimants (102), au moins un aimant du second ensemble d'aimants présentant une polarité différente de celle d'au moins un autre aimant du second ensemble d'aimants ; et
le premier corps de connecteur est conçu pour se connecter au second corps de connecteur, le premier corps de connecteur étant aligné en rotation avec le second corps de connecteur dans une position de rotation spécifique de sorte que le premier ensemble d'aimants se couple magnétiquement au second ensemble d'aimants de sorte que les forces d'attraction/répulsion entre le premier ensemble d'aimants et le second ensemble d'aimants dictent l'alignement en rotation du premier corps de connecteur et du second corps de connecteur.

2. Système de champ opératoire selon la revendication 1, dans lequel :
le premier corps de connecteur présente une face (70) conçue pour entrer en contact avec le premier côté du champ opératoire ;
le second corps de connecteur présente une face (72) conçue pour entrer en contact avec le second côté du champ opératoire ; et
la face du premier corps de connecteur présente une forme complémentaire à la face du second corps de connecteur.

3. Système de champ opératoire selon la revendication 2, dans lequel la face du premier corps de connecteur et la face du second corps de connecteur sont chacune planes.

4. Système de champ opératoire selon la revendication 2, dans lequel la face du premier corps de connecteur et la face du second corps de connecteur sont chacune non-planes de sorte que, lorsque le champ opératoire est positionné entre le premier corps de connecteur et le second corps de connecteur, le matériau de champ opératoire est plié dans des plans différents s'étendant à travers le connecteur.

5. Champ opératoire selon la revendication 4, dans lequel :
la face du premier corps de connecteur comporte une ou plusieurs saillies (80) et/ou un ou plusieurs évidements ; et
la face du second corps de connecteur comporte un ou plusieurs évidements complémentaires (82) et/ou des saillies conçues pour s'adapter dans la ou les saillies et/ou évidements définis sur la face du premier corps de connecteur.

6. Système de champ opératoire selon la revendication 4 ou 5, dans lequel :
la face du premier corps de connecteur comporte une pluralité de saillies et d'évidements alternés ;
et la face du second corps de connecteur comporte une pluralité d'évidements et de saillies complémentaires conçus pour s'adapter dans les saillies et les évidements alternés du premier corps de connecteur.

7. Système de champ opératoire selon l'une quelconque des revendications 1 à 6, dans lequel la tête du dispositif de suivi de patient comprend une tige, et l'extrémité distale de la tête est définie par une extrémité distale de la tige.

8. Système de champ opératoire selon l'une quelconque des revendications 1 à 7, dans lequel la tête du dispositif de suivi de patient comprend un réseau de suivi (64) portant une pluralité de marqueurs de suivi (24).

9. Système de champ opératoire selon l'une quelconque des revendications 1 à 8, dans lequel la base du dispositif de suivi de patient comprend une tige, et l'extrémité proximale de la base est définie par une extrémité proximale de la tige.

10. Système de champ opératoire selon la revendication 9, dans lequel la tige est conçue pour être insérée dans le patient.

11. Système de champ opératoire selon la revendication 10, dans lequel la tige est conçue pour être insérée par voie percutanée dans un os du patient.

12. Système de champ opératoire selon l'une quelconque des revendications 1 à 11, dans lequel le système de reconnaissance de position chirurgicale comprend un système optique de reconnaissance de position chirurgicale.

13. Système de champ opératoire selon l'une quelconque des revendications 1 à 12, dans lequel le matériau de champ opératoire comprend un matériau polymère.

14. Système de champ opératoire selon l'une quelconque des revendications 1 à 13, dans lequel le matériau de champ opératoire définit une feuille continue de matériau dépourvue de toute ouverture dans la région entre le premier corps de connecteur et le second corps de connecteur.
